# EUROPEAN PATENT SPECIFICATION

(11) **EP 0 935 139 B1**
(45) Date of publication and mention of the grant of the patent: **17.11.2004**
(21) Application number: 97941192.3
(22) Date of filing: 18.09.1997
(51) Int. Cl.: G01N 33/533, G01N 33/574

(54) **IMMUNOHISTOCHEMICAL STAINING COMPOSITION**
ZUSAMMENSETZUNG ZUR IMMUNOHISTOCHEMISCHEN ANFÄRBUNG
COMPOSITION DE COLORATION IMMUNOHISTOCHIMIQUE

(30) Priority: 19.09.1996 JP 24678296; 26.12.1996 JP 34788696; 28.02.1997 JP 4551697
(43) Date of publication of application: 11.08.1999
(73) Proprietor: DAIICHI PURE CHEMICALS CO., LTD., Chuo-ku Tokyo 103-0027 (JP)
(72) Inventor: SHIBAMURA, Seiichi, Daiichi Pure Chemicals Co.Ltd, Chuo-ku Tokyo 103 (JP); ITO, Susumu, Tokushima-shi Tokushima 770 (JP); TAKESAKO, Kazuhiro, 56-10, Koga, Kumamoto 861-22 (JP); IRIMURA, Tatsuro, Setagaya-ku Tokyo 154 (JP)
(74) Representative: Zimmermann & Partner
(86) International application number: PCT/JP1997/003306
(87) International publication number: WO 1998/012560

(56) References cited:
- WO-A-94/25853
- WO-A-96/02839
- JP-A- 9 127 115
- US-A- 5 521 061
- US-A- 5 547 836
- FOLLI S ET AL: "ANTIBODY-INDOCYANIN CONJUGATES FOR IMMUNOPHOTODETECTION OF HUMAN SQUAMOUS CELL CARCINOMA IN NUDE MICE" CANCER RESEARCH, AMERICAN ASSOCIATION FOR CANCER RESEARCH, BALTIMORE, MD, US, vol. 54, 15 May 1994 (1994-05-15), pages 2643-2649, XP000646876 ISSN: 0008-5472
- ITO, SUSUMU ET AL: "Development of agents for reinforcement of fluorescence on near-infrared ray excitation for immunohistological staining" BIOORG. MED. CHEM. (1998), 6(5), 613-618 , XP001032824

## Description

The present invention relates to a composition for immunohistochemical staining. More specifically, the present invention relates to a composition for immunohistochemical staining having excellent fluorescence intensity which contains a diagnostic marker comprising a labeling compound, which is excited by irradiation with near infrared rays or far infrared rays that rarely cause histological damage and emits fluorescence, bound with an antibodies or other that specifically recognizes tumor cells and the like.

### Background Art

In recent years, endoscopic diagnosis has easily been conducted with the spread of electronic endoscopes. It becomes possible to infallibly find stomach cancer or large bowel cancer as initial cancers. However, as far as the diagnosis of microcarcinoma is concerned, almost the same levels of diagnostic performance are achieved by an electronic endoscope and an ordinary endoscope. The fact means that new diagnostic methods, in which electronic endoscopes function efficiently, have not yet been established. If microlesions such as those not recognizable by an ordinary endoscope can be marked with a labeling antibody that is detectable under electronic endoscopy, it may be possible to easily detect micrlesions by visualizing through a processing using a computer. However, such method has not yet been practically developed.

In order to establish a method utilizing an electronic endoscope such as described above, it is necessary to conduct direct staining of a living tissue by an immunohistochemical staining method. Staining for fixed specimens are already established techniques. However, a staining for non-fixed specimens has not yet become available to those skilled in the art. For example, an immunostaining method for non-fixed specimens was reported [Shikoku Igaku Zasshi (Shikoku Medical Journal), 29, 180, 1987]; however, no immunostaining method that utilizes near infrared ray has been reported which is applied to an excised fresh specimen or a living tissue, per se.

In addition, a diagnostic marker that is detectable under electronic endoscopy, e.g., a labeled antibody, is also required for the aforementioned diagnostic method. Diagnostic markers are known in which an antibody is bound to a labeling compound that emits fluorescence as ultraviolet and visible light when excited with ultraviolet rays. The markers have been commonly used for the detection of cancer cells or cancer tissues that are present in tissues isolated from living bodies. However, methods utilizing fluorescent diagnostic markers that needs excitation with ultraviolet rays cannot be applied to living bodies, because ultraviolet rays may cause damages on living tissues and DNAs. No diagnostic marker that can be directly applied to a living body has been known so far.

It is known that indocyanine green (ICG) has unique absorption properties and emits fluorescence under infrared ray endoscopy. Clinical cases were reported in which indocyanine green was applied when an infrared ray endoscope is used (Gastroenterological Endoscopy, 34, pp.2287-2296, 1992; and Gastrointestinal Endoscopy, 40, pp.621-2;628, 1994). However, in these cases, ICG was intravascularly administered. Furthermore, fluorescent dyes, including indocyanine green as a typical example, have generally high hydrophobicity and are absorbed rapidly when they, per se, are administered into intestinal tract. For this reason, attempts have been made to increase their water-solubility by introducing hydrophilic groups, e.g., sulfonyl group, into ring structures or side chain moieties, and thereby improve measurement efficiency and eliminate the problem of toxicity after absorption (as a review of the background art described above, see, for example, Kina K., Section 2: Dyes for Clinical Examination (Diagnosis), In "The Latest Applied Technology of Functional Dyes", Ed. by M. Irie, CMC Co., Ltd., 1996 and the like).

Through synthesis of various indocyanine green derivatives, the inventors of the present invention succeeded in preparing indocyanine green derivatives that emit fluorescence under excitation with near infrared rays and far infrared rays. They also found that a diagnostic marker that is directly applicable to living bodies can be prepared by reacting the aforementioned indocyanine green derivative, as a labeling compound, with an anti-cancer antigen-antibody and the like, and that the diagnostic marker as mentioned above is useful for a direct staining of a living tissue by an immunohistochemical staining method. The inventors filed a patent application directed to these inventions (Japanese Patent Application No. Hei 7-12283/1995).

In addition, the inventors earnestly conducted researches to provide diagnostic markers having excellent water solubility. As a result, they found that, among the aforementioned indocyanine green derivatives, compounds that can form an intramolecular ion pair (a zwitterion) may have reduced water solubility due to the decrease of molecular ionic property after the formation of the intramolecular ion pair, whilst these derivatives do not form intramolecular ion pair when treated with sodium iodide or other, and whole molecular ionic properties are maintained and thereby water solubilities are remarkably increased. The inventors also filed a patent application directed to these inventions (Japanese Patent Application No. Hei 7-223613/1995).

However, further researches on diagnostic markers containing a fluorescent label compound such as indocyanine green derivatives bound to an antibody revealed that the fluorescence intensity of the diagnostic markers is reduced to about one tenth of that of the indocyanine green derivatives (compounds for labeling), per se, before the binding. When immunohistochemical staining is performed in vivo by using the aforementioned diagnostic markers to detect microtissues such as cancer tissues, the above problem may likely be overcome by using a fluorescence detection apparatus having a markedly higher sensitivity compared to conventional ones. However, development and practicing of apparatuses having high performance may require enormous efforts and economical investment. On the other hand, if an agent for enhancing fluorescence intensity is provided that specifically acts on a diagnostic marker comprising a fluorescent labeling compound bound to an antibody and enhances its fluorescence intensity, stained tissues may possibly be detected surely and conveniently by using a currently available apparatus.

### Description of the Invention

An object of the present invention is to provide a substance acting on a diagnostic marker comprising a fluorescent labeling compound such as indocyanine green derivatives bound to an antibody, and enhancing its fluorescence intensity. Another object of the present invention is to provide a composition for immunohistochemical staining having remarkably enhanced fluorescence intensity to use the aforementioned diagnostic marker for immunohistochemical staining in living bodies as defined by the appended claims.

The inventors of the present invention made various efforts to achieve the foregoing objects, and as a result, they found that a substance selected from the group consisting of glycerophospholipids, for example, acylglycerol phosphates such as dimyristoylphosphatidic acid and distearoylphosphatidic acid, acylglycerolphosphocholine such as distearoylphosphatidylcholine and the like; fatty acids such as stearic acid; and surfactants consisting of saccharide derivatives such as octyl glucoside can remarkably enhance the fluorescence intensity of diagnostic markers containing a fluorescent labeling compound such as indocyanine green derivatives bound to an antibody. They also found that a composition containing such substance and the diagnostic marker is extremely useful as a composition for the immunohistochemical staining applicable to living bodies, and a stable composition having excellent solubility can be provided by using a surfactant consisting of saccharide derivative such as octyl glucoside as an essential component in the aforementioned composition. The present invention was completed on the basis of these findings.

The present invention thus provides a composition for immunohistochemical staining characterized in that said composition contains a diagnostic marker comprising an antibody bound with a fluorescent functional group derived from an indocyanine green derivative together with a a surfactant consisting of a saccharide derivative selected from octylglucoside, heptylglucoside, octylthioglucoside or heptylthioglucoside. According to preferred embodiments of the present invention, the composition additionally contains a glycerophospholipid or a fatty acid wherein the glycerophospholipid is an acylglycerol phosphate; the above composition wherein the acylglycerol phosphate is a 1,2-diacyl-sn-glycerol 3-phosphate containing two C₁₀₋₂₀ fatty acid residues; the above composition wherein the 1,2-diacyl-sn-glycerol 3-phosphate is dimyristoylphosphatidic acid or distearoylphosphatidic acid; the above composition wherein the glycerophospholipid is an acylglycerol phosphocholine; the above composition wherein the acylglycerol phosphocholine is a 1,2-diacyl-sn-glycerol 3-phosphocholines containing two C₁₀₋₂₀ fatty acid residues; the above composition wherein the 1,2-diacyl-sn-glycerol 3-phosphocholine is distearoylphosphatidylcholine; and the above composition wherein the surfactant is octyl glucoside.

According to the present invention, there are further provided the above composition wherein the fluorescent functional group is a functional group derived from indocyanine green-N-hydroxysulfosuccinimide ester; and the above composition wherein the antibody is an anti-cancer antigen antibody.

According to another aspect of the present invention, there is further provided; a method for immunohistochemically staining a living tissue by using a composition according to claims 1-11, and a method for immunohistochemically diagnosing a tumor by using a composition according to claims 1-11.

### Brief Explanation of the Drawings

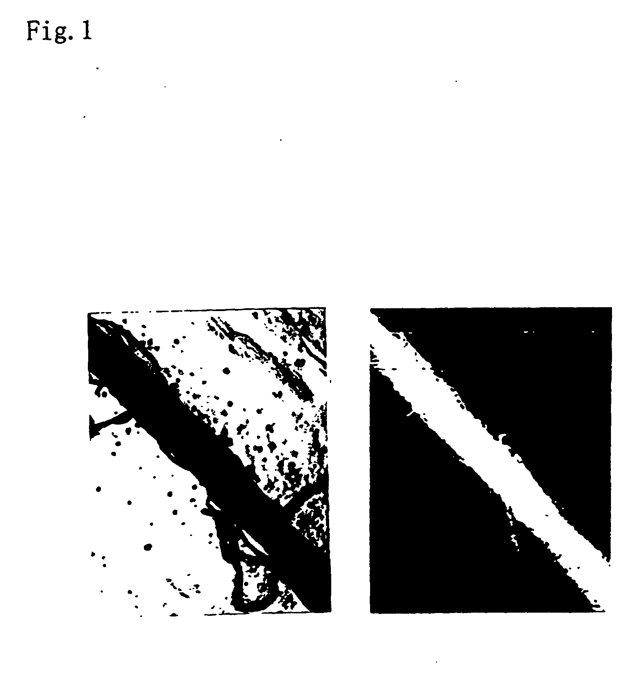

Fig. 1 shows photographs of fiber tissues of cotton gauze thread dropped with a composition of the present invention taken under microscope with ordinary light and infrared irradiation. In the figure, (a) shows the result obtained under ordinary light, and (b) shows the result obtained under infrared irradiation.

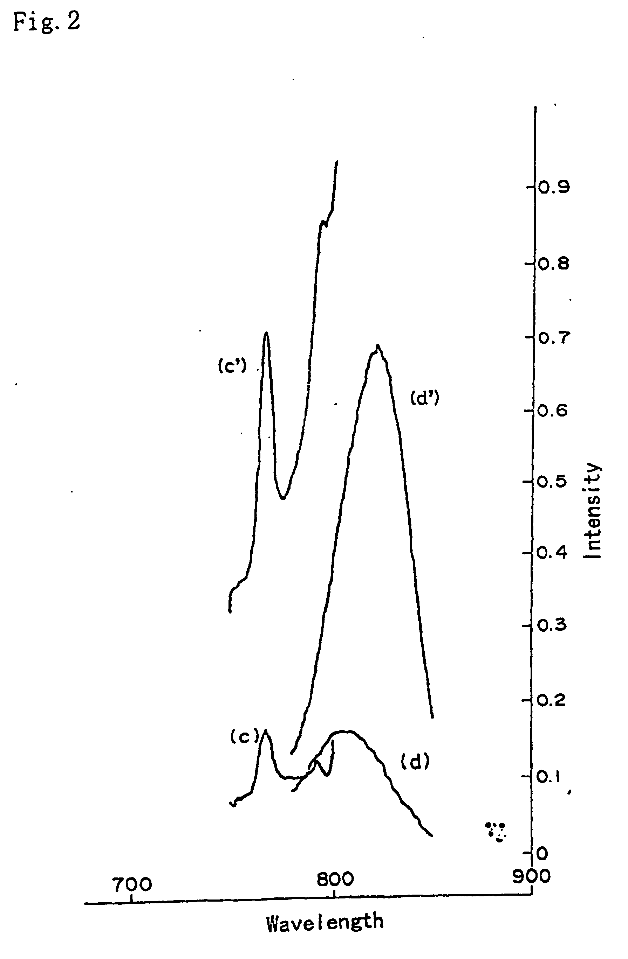

Fig. 2 shows changes of absorption and fluorescence spectra before and after, dimyristoylphosphatidic acid and octyl glucoside as the agent for enhancing fluorescence intensity was added to mouse anti-mucin antibody bound with ICG-sulfo-OSu. In the figure, (c) shows absorption spectrum before the addition of the agent for enhancing fluorescence intensity; (c') shows absorption spectrum of the composition of the present invention after the addition of the agent for enhancing fluorescence intensity; (d) shows fluorescence spectrum before the addition of the agent for enhancing fluorescence intensity; and (d') shows fluorescence spectrum of the composition of the present invention after the addition of the agent for enhancing fluorescence intensity.

### Best Mode for Carrying Out the Invention

The composition of the present invention is used for immunohistochemical staining, and characterized to contain a diagnostic marker comprising an antibody bound with a fluorescent functional group derived from an idocyanine green derivative together with a surfactant consisting of a saccharide derivative selected from octylglucoside, heptylglucoside, octylthioglucoside or heptylthioglucoside. The composition according to a preferred embodiment of the present invention is characterized to further contain a substance selected from the group consisting of glycerophospholipids and fatty acids.

As used herein, the term "glycerophospholipid" means a phospholipid containing glycerol as a basic structure, and more specifically, it means a phospholipid containing glycerol phosphate (also referred to as glycerophosphate). Typical examples of the glycerophospholipid include, for example, acylglycerol phosphocholines such as 1,2-diacyl-sn-glycerol 3-phosphocholine (phosphatidylcholine); acylglycerol phosphoethanolamines such as 1,2-diacyl-sn-glycerol 3-phosphoethanolamine (phosphatidylethanolamine); acylglycerol phosphoserines such as 1,2-diacyl-sn-glycerol 3-phospho-L-serine (phosphatidylserine); phosphatidylinositols; acylglycerol phosphates such as 1,2-diacyl-sn-glycerol 3-phosphate (phosphatidic acid); diphosphatidylglycerol and the like.

The glycerol phosphate constituting the glycerophospholipid mentioned above may be any of the isomers thereof, i.e., glycerol 1-phosphate, glycerol 2-phosphate, and glycerol 3-phosphate. Although its stereochemistry is not particularly limited, glycerophospholipids comprising naturally occurring sn-glycerol 3-phosphate (L-α-glycerol phosphate) may preferably be used (glycerol 3-phosphate is represented by the formula: HOCH₂-CH(OH)-CH₂O-PO₃H₂). Among the glycerophospholipids mentioned above, those preferably used in the composition of the present invention are acylglycerol phosphates and acylglycerol phosphocholines.

As herein used, the term "acylglycerol phosphate" means a monofatty acid ester or difatty acid ester of glycerol phosphate, i.e., a compound represented by the following formula: A¹OCH₂-CH(OA²)-CH₂O-PO₃H₂ wherein A¹ and A² independently represent hydrogen atom or an acyl group as a fatty acid residue, provided that A¹ and A² do not simultaneously represent hydrogen atom. In the above formula, the fatty acid residue, i.e., a group formed by removing a hydroxyl group represented as - OH from fatty acids represented as A¹OH and/or A²OH, that constitutes the above monofatty acid ester or difatty acid ester may be a fatty acid residue having about 8-22 carbon atoms (C₈₋₂₂), preferably about 10-20 carbon atoms (C₁₀₋₂₀). For example, residues derived from fatty acid having 10, 12, 14, 16, or 18 carbon atoms are preferably used.

These fatty acid residues may be linear or branched, and may be saturated or unsaturated. The fatty acid residues constituting the difatty acid ester may be the same or different. As the acylglycerol phosphate, for example, distearoyl ester of sn-glycerol 3-phosphate (distearoylphosphatidic acid), dimyristoyl ester of sn-glycerol 3-phosphate (dimyristoylphosphatidic acid) and the like can suitably be used. The acylglycerol phosphate may be used as an alkali salt. Examples of such an alkali salt include, for example, sodium salts and potassium salts. Among them, sodium salts of distearoylphosphatidic acid and dimyristoylphosphatidic acid are preferred.

As used herein, the term "acylglycerol phosphocholine" means a monofatty acid ester or difatty acid ester of glycerol phosphocholine, i.e., a compound represented by the following formula: A³OCH₂-CH(OA⁴)-CH₂O-PO(OH·)OCH₂CH₂N⁺(CH₃)₃ wherein A³ and A⁴ independently represent hydrogen atom or an acyl group as a fatty acid residue, provided that A³ and A⁴ do not simultaneously represent hydrogen atom. In the above formula, the fatty acid residue, i.e., a group formed by removing a hydroxyl group represented as -OH from fatty acids represented as A³OH and/or A⁴OH, that constitutes the above monofatty acid ester or difatty acid ester may be a fatty acid residue having about 8-22 carbon atoms (C₈₋₂₂), preferably about 10-20 carbon atoms (C₁₀₋₂₀). For example, residues derived from fatty acid having 10, 12, 14, 16, or 18 carbon atoms are preferably used. These fatty acid residues may be linear or branched, and may be saturated or unsaturated. The fatty acid residues constituting the difatty acid ester may be the same or different. As the acylglycerol phosphate, for example, distearoyl ester of sn-glycerol 3-phosphocholine (distearoylphosphatidylcholic acid) and the like can be suitably used.

As the fatty acid, for example, fatty acids having about 8-22 carbon atoms (C₈₋₂₂), preferably about 10-20 carbon atoms (C₁₀₋₂₀) may be used. For example, fatty acids having 10, 12, 14, 16, or 18 carbon atoms are preferred. These fatty acid may be linear or branched, and may be saturated or unsaturated. For example, stearic acid and the like can be suitably used. The surfactant, which is a saccharide derivative, is not particularly limited so long as it does not substantially denature proteins, and has low stimulation against living tissues such as skins and mucosa. For example, octyl glucoside, heptyl glucoside, octyl thioglucoside, heptyl thioglucoside can be used.

The saccharide derivatives mentioned above, per se, have fluorescence intensity enhancing effect; however, the saccharide derivative may preferably be used in combination with a substance selected from the group consisting of glycerophospholipids and fatty acids when the substance selected from the group consisting of glycerophospholipids and fatty acids is slightly soluble in water. A water-insoluble substance such as acylglycerol phosphate can be solubilized in water by the aid of the surface activating property of the saccharide derivative, which may sometimes facilitate the preparation of the composition, and remarkably improve the stability of the product. Synergistic fluorescence intensity enhancing effect may also be expected by using a saccharide derivative and a substance selected from the group consisting of glycerophospholipids and fatty acids. For example, a composition containing octyl glucoside and distearoylphosphatidic acid and a composition containing octyl glucoside and dimyristoylphosphatidic acid are preferred embodiments of the present invention.

The content amount of the aforementioned fluorescence intensity enhancing agent is not particularly limited, and the amount may be appropriately chosen depending on a type of the diagnostic marker used, a type of excitation light and other. When the aforementioned saccharide derivative and a substance selected from the group consisting of glycerophospholipids and fatty acids are used in combination, the saccharide derivative can be used in an amount suitable for solubilizing the substance selected from the group consisting of glycerophospholipids and fatty acids. For example, use of the saccharide derivative at a concentration near the critical micelle concentration (about 25 mM for octyl glucoside) may sometimes be preferred.

The diagnostic marker contained in the composition of the present invention is not particularly limited, so far that the marker comprises an antibody bound with a fluorescent functional group and can be used for immunohistochemical staining. For example, diagnostic markers may preferably be used which emit fluorescence having a wavelength of 780 nm or more, preferably 780-840 nm or more when irradiated with an excitation light having a wavelength of 600-800 nm. Among them, those markers are most preferably used for the composition of the present invention which can be excited by near infrared rays and far infrared rays, and emit fluorescence of 810 nm or more, preferably 820 nm or more, because such markers will not damage living tissues and DNAs during diagnosis. Highly water-soluble diagnosis markers are also preferably used. The composition of the present invention may contain one or more diagnosis markers.

As herein used, the term "fluorescent functional group" means a chemical structure which is a fluorescent partial structure derived from fluorescent labeling compound and binds to an antibody through a reaction between the labeling compound and the antibody. As the labeling compound used for binding a fluorescent functional group to an antibody, for example, indocyanine green derivatives can be used. As preferred labeling compounds, for example, indocyanine green-N-hydroxysuccinimide ester (ICG-OSu), indocyanine green-N-hydroxysulfosuccinimide ester (ICG-sulfo-OSu) described in Biooraganic & Medicinal Chemistry Letters, 5(22), pp.2689-2694, 1995 and other can be used. By using these labeling compounds, the whole ring structure of the indocyanine green derivatives as a fluorescent functional group can be readily attached to an antibody.

The fluorescent functional group and antibody may be bound directly to each other, or alternatively, they may be bound by means of a linker or a protein such as albumin. One or more, preferably about 10 or more of the aforementioned fluorescent functional groups can be attached to a protein such as albumin. Moreover, an antibody may be also readily introduced. Diagnostic markers utilizing such protein are preferred embodiments of the present invention.

Among diagnostic markers which may be contained in the composition of the present invention, preferred diagnostic markers include:
① diagnostic markers which comprise (a) an antibody; and (b) a fluorescent functional group which is bound to the antibody and represented by the following formula (I): 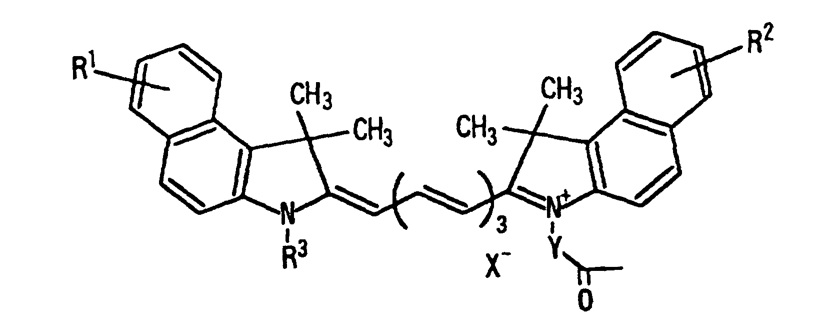 wherein R¹ and R² independently represent hydrogen atom, an alkyl group, an aryl group, an alkoxyl group, or a sulfonic acid group; R³ represents an alkyl group, a sulfonic acid-alkyl group, or an amino-substituted alkyl group; X⁻ represents an anion species, if required; Y represents a C₁-C₁₀ alkylene group or a C₁-C₁₀ alkylene group containing one or more atoms selected from the group consisting of oxygen atom, nitrogen atom, and sulfur atom; and
② diagnostic markers which comprise (a) an antibody; and (b) a fluorescent functional group which is bound to the antibody and represented by the following formula (II): 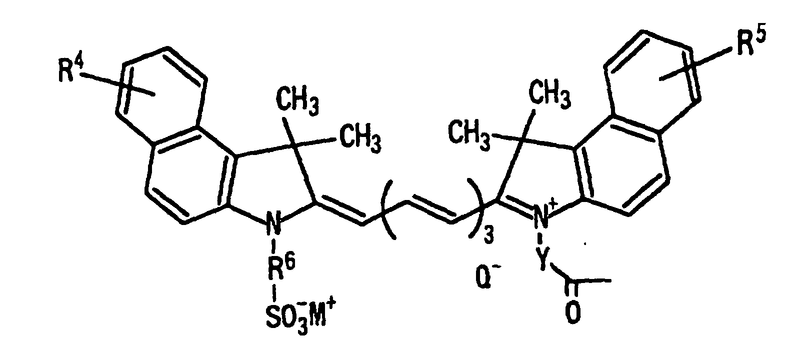 alkoxyl group, or a sulfonate group are preferred.

R³ represents an alkyl group, a sulfonic acid-alkyl group, or an amino-substituted alkyl group. As the alkyl group in these groups, for example, those mentioned above may be used. A sulfonic acid group of the sulfonic acid-alkyl group or an amino group of the amino-substituted alkyl group may substitute at any position of an alkyl group. For example, those with substitution at the terminal of an alkyl group may preferably be used.

The sulfonic acid group and the amino group may form salts independently or with each other. For example, those wherein the sulfonic acid groups form sodium salts or potassium salts, those wherein the amino groups form salts such as ammonium halides, or those wherein the amino groups form quaternary amines are preferred. In addition, substituted or non-substituted amino groups may be used as the amino group. Examples of the sulfonic acid-alkyl group and the amino-substituted alkyl group include sulfonic acid-methyl group (-CH₂SO₃H), sulfonic acid-ethyl group, aminomethyl group, aminoethyl group, methylaminoethyl group, and salts thereof.

In the fluorescent functional group represented by the formula (I), X⁻ represents an anion species, if required, such as halogen ion, acetate ion, perchlorate ion, and carbonate ion. The anion species represented by X⁻ acts to cancel positive charge on the nitrogen atom in the ring that is substituted with Y-CO- group, so that the fluorescent functional group represented by the formula (I) as a whole is maintained neutral. Therefore, for example, when one of the groups R¹, R², and R³ in the fluorescent functional group represented by the formula (I) is an anionic group, X⁻ may be sometimes not required, because the negative charge of the group cancel the positive charge on the quaternary nitrogen atom of the ring structure so as to form an intramolecular zwitterion. On the other hand, when any one of R¹ and R² is a sulfonic acid group and R³ is an amino-substituted alkyl group, charges between these groups may be balanced, and as a result, X⁻ may sometimes be required.

In the fluorescent functional group represented by the above formula (II), R⁴ and R⁵ independently represent hydrogen atom, an alkyl group, an alkoxyl group, or a sulfonate group. Each of R⁴ and R⁵ may substitute on the phenyl group at any position. As the alkyl group and the alkoxyl group, those mentioned above may be used. The sulfonate group (-SO₃⁻M⁺, wherein M⁺ represents an alkali metal ion that may be the same as or different from M⁺ as a counter ion for Q⁻) may be, for example, sodium sulfonate group or potassium sulfonate group.

R⁶ represents a straight- or branched-alkylene group. For example, a straight- or branched-lower alkylene group having 1 to 6 carbon atoms, preferably those having 2 to 5 carbon atoms, and more preferably trimethylene group, tetramethylene group, or pentamethylene group may be used. The -SO₃· group substituting on R⁶ may bind to the alkylene group at any position. For example, those with substitution at the terminal of an alkylene group may preferably be used. More specifically, a group represented by -(CH₂)ₖ-SO₃- wherein k is an integer of from 2 to 4 and the like are preferred as R⁶-SO₃·.

M⁺ represents an alkali metal ion. As the alkali metal ion, sodium ion or potassium ion may preferably be used. Q⁻ represents a halogen ion, perchlorate ion, or thiocyanate ion. Preferably, chloride ion, bromide ion, iodide ion or the like may be used. Among them, iodine ion is particularly preferred. Although not intended to be bound by any specific theory, the aforementioned fluorescent functional group has positive charge on the nitrogen atom on which -Y-CO- group substitutes (represented as N⁺ in the above formula) and negative charge derived from R³-SO₃·. Where an alkali metal salt represented by M⁺Q⁻ co-exists, ionic bonds are formed respectively between the positive charge on the nitrogen atom (represented by N⁺ in the above formula) and Q⁻, as well as between R³-SO₃· and M⁺. As a result, formation of an intramolecular pair ions is prevented, and the ionic property of the whole molecule is maintained and water solubility is remarkably increased.

In the above formulas (I) and (II), Y represents a straight- or branched-alkylene group having 1 to 10 carbon atoms, preferably a straight- or branched-alkylene group having 3 to 5 carbon atoms, and more preferably trimethylene group, tetramethylene group or pentamethylene group. Alternatively, Y represents a straight- or branched-alkyl group having 1 to 10 carbon atoms which contains one ore more atoms selected from the group consisting of oxygen atom, nitrogen atom, and sulfur atom. As the group represented by -Y-CO-, for example, -CH₂-CO-; -(CH₂)₂-CO-; -(CH₂)₃-CO-; -(CH₂)₄-CO-; -(CH₂)₅-CO-; -CH₂-CO-NH-(CH₂)₆-CO-; -(CH₂)₂-CO-NH-(CH₂)₅-CO-; -(CH₂)₃-CO-NH-(CH₂)₅-CO-; -(CH₂)₄-CO-NH-(CH₂)₆-CO-; -CH₂-CO-NH-(CH₂)₅-CO-NH-(CH₂)₂-CO-; -(CH₂)₄-CO-(N,N'-piperadinyl)-(CH₂)₂-CO- ("N,N'-piperadinyl" means that a piperazine is substituted with -(CH₂)₄-CO- at the 1-position and with -(CH₂)₂-Z group at the 4-position, and similarly used hereinafter in the specification.), -CH₂-CO-NH-(CH₂)₅-CO-(N,N'-piperadinyl)-(CH₂)₂-CO- and the like may be utilized.

The carbonyl group of-Y-CO- group may be bound to an antibody by means of an additional group such as a straight- or branched-alkylene group having 1 to 10 carbon atoms; a straight- or branched-alkylene group having 1 to 10 carbon atoms that contains one ore more atoms selected from the group consisting of oxygen atom, nitrogen atom, and sulfur atom; or -NH-NH- group. The aforementioned preferred diagnostic markers are specifically disclosed in the specifications of the Japanese Patent Application Nos. (Hei) 7-12283/1995 and (Hei) 7-223613/1995, and detailed explanation of the preparing methods thereof are also given in the specifications. Those skilled in the art will readily prepare the aforementioned preferred diagnostic markers in view of the disclosures. In addition, labeling compounds used for binding the fluorescent functional groups represented by the aforementioned formulas (I) and (II) to antibodies are also disclosed in the aforementioned specifications. In the fluorescent functional groups represented by the aforementioned formulas (I) and (II), the positive charge on nitrogen atoms (represent by N⁺ in the above formulas) is indicated in a fixed manner on one nitrogen atom of the ring structure for convenience sake. However, it will be readily understood by those skilled in the art that the positive charge can move to another nitrogen atom through conjugated double bonds.

As the antibody that binds to the aforementioned fluorescent functional group, antibodies recognizing various antigens, such as antibodies highly specific to cancers, may be used. As the antibodies, for example, anti-cancer antigen-antibodies may be used which specifically bind to cancer cells or cancer tissues, preferably to early cancer cells or early cancer tissues. More specifically, anti-tumor antibodies, anti-mucin antibodies, and anti-sugar chain antibodies relating to stomach which specifically react with CEA, AFP, CA19-9, NSE, DU-PAN-2, CA50, SPan-1, CA72-4, CA125, HCG, p53, STN (sialyl Tn antigen), c-erbB-2 proteins and other, and anti-tumor antibodies specifically reacting with tumor antigens of esophageal carcinoma, large bowel cancer, rectum cancer, skin cancer, uterus cancer and other can be utilized. Anti-pathogenic protein-antibodies, anti-tumor antigen antibodies and the like which are bound with an amplification system such as avidin and biotin may also be used. However, the antibodies explained above are given only as examples, and antibodies which can be used for the diagnosis marker are not limited to those mentioned above. Any antibodies may be used so long as they have substantial property to bind specifically to target cells or target tissues that are objects of examination and diagnosis.

The antibody contained in the diagnostic marker specifically binds to cancerous antigens or other, and as a result, lesions such as cancer cells or cancer tissues are immunologically stained with the diagnostic marker. Then, the lesions that emit fluorescence can be recognized under irradiation with near infrared rays or far infrared rays using an infrared laser or other. The fluorescence intensity enhancing agent contained in the composition of the present invention enhances fluorescence intensity of the diagnostic marker by several times to several tens of times, and accordingly, it becomes possible to easily observe the lesions by fluorescence detectors.

The composition of the present invention is generally provided in the form of aqueous compositions or as compositions in a solid state such as pulverized powders and lyophilized powders. Preferred aqueous compositions containing a saccharide derivative and acylglycerol phosphate as the fluorescence intensity enhancing agent can be prepared, for example, by dissolving the surfactant such as octyl glucoside in an aqueous medium such as physiological saline and phosphate buffered saline as required, and then adding sodium salt of distearoylphosphatidic acid, for example, to the medium and dissolving at room temperature to about 60°C, preferably about 50°C, more preferably about 40°C, followed by adding a solution obtained by dissolving a diagnostic marker in an aqueous medium such as physiological saline and phosphate buffered saline to the above solution and mixing the mixture. However, the preparation method of the composition of the present invention is not limited to the method mentioned above, and it should be understood that appropriate methods can be chosen by those skilled in the art. The composition of the present invention can be prepared also as a composition in the form of lyophilized powder by lyophilizing the aforementioned aqueous composition.

Alternatively, an aqueous solution containing the fluorescence intensity enhancing agent, such as an aqueous solution containing an acylglycerol phosphate and a surfactant as required, may be prepared separately from an aqueous solution containing a diagnostic marker, and then mixing both of the solutions upon use, i.e., just before diagnosis, to prepare the composition of the present invention. It is further possible to perform diagnosis by administering an aqueous solution containing the diagnostic marker, and then separately administering an aqueous solution containing the fluorescence intensity enhancing agent.

As pharmacologically and pharmaceutically acceptable additives for the preparation of the composition of the present invention, for example, excipients, disintegrators or disintegrating aids, binders, lubricants, coating agents, coloring materials, diluents, base materials, solubilizers or dissolving aids, isotonicities, pH modifiers, stabilizers, propellants, thickeners and the like may be used. For example, excipients such as glucose, lactose, D-mannitol, starch, or crystalline cellulose; disintegrators or disintegrating aids such as carboxymethylcellulose, starch, or carboxymethylcellulose calcium; base materials such as Vaseline, liquid paraffin, polyethylene glycol, gelatin, china clay, glycerin, purified water, or hard fat; isotonicities such as glucose, sodium chloride, D-mannitol, or glycerin; pH modifiers such as inorganic acids, organic acids, inorganic bases, or organic bases; substances that increase stability such as vitamin A, vitamin E, or coenzyme Q may be added.

As an example of a method for utilizing the composition for immunohistochemical staining of the present invention as a diagnostic agent, an examination process using an infrared ray endoscope will be explained. A focal portion that emit fluorescence can be detected by staining a lesional portion by endoscopically splaying or applying the aforementioned diagnostic agent (at a concentration of about 0.1 to 1,000 mg/ml) to a tissue that is suspected to involve focal portions, conducting appropriate washings to remove excess diagnostic agent from the tissue, and then irradiating the tissue with near infrared rays or far infrared rays, more specifically, a light having a wavelength of, for example, 600-800 nm, preferably about 768 nm, more preferably a laser excitation light. Although the composition of the present invention is characterized in that it can be directly applied to living bodies and exhibit excellent fluorescence intensity, it should be understood that the methods of using the composition of the present invention are not limited to those applied to living bodies, and that the composition is also applicable to fixed specimens such as paraffin embedded preparations.

The detection of fluorescence can be carried out, for example, by means of infrared ray endoscope, infrared ray microscope and other. For example, a filter having given transmission properties, more specifically, one filter or two or more filters in combination chosen from filters having shielding property against the excitation light and filters for detecting fluorescence may be used. Where endoscopic examination is carried out by applying the composition of the present invention to a living body, an endoscope having a magnification of about 10 to 1,000 may be used. For example, an infrared ray endoscope having a microscopic level of magnification may preferably be used. The endoscope may be provided with a means for spraying or applying the composition of the present invention and means for washing.

Where the composition of the present invention is applied to tissues or specimens isolated from living bodies, an infrared ray microscope can be used for the detection of fluorescence. Image analysis may also be conducted by observing preparations under normal light to recognize stained portions, and then taking photographs using an infrared film in a darkroom under infrared rays, or alternatively, recording in videotapes, for example, as a recording medium.

### EXAMPLES

The present invention will be further explained more specifically by referring to the following examples. However, the scope of the present invention is not limited to the following examples.

### Example 1

By using ICG-sulfo-OSu (Biooraganic & Medicinal Chemistry Letters, 5(22), pp.2689-2694, 1995) as an indocyanine green derivative, a diagnostic marker comprising mouse anti-CEA antibody carrying approximately 16 molecules of ICG-sulfo-OSu per one molecule of the antibody was prepared according to the method described in the literature. The diagnostic marker was stored in frozen condition just before use. The diagnostic marker was dissolved in PBS⁻ containing 37.5 mM of octyl glucoside, and absorption spectrum and fluorescence spectrum were measured. As a result, the marker was found to have the maximum absorption wavelength of 802.8 nm, molar absorption coefficient of 3.90× 10⁵ M⁻¹·cm⁻¹ (slit width: 0.2 nm, reference: PBS⁻ containing 37.5 mM of octyl glucoside, measured at ambient temperature); excitation wavelength of 768 nm, and fluorescence wavelength of 820 nm (slit width on the excitation light side: 5.0 nm, slit width on the fluorescence side: 5.0 nm).

Octyl glucoside (Dojindo Laboratories, 880 mg) was carefully added to phosphate buffered saline (10 ml, pH 7.4) and dissolved with stirring on a water bath at 40°C. Sodium distearoylphosphatidate (148.8 mg) was added to the resulting solution and dissolved with stirring over a water bath at 60°C, and then the solution was added with phosphate buffered saline up to a final volume of 20 ml. The solution was returned to room temperature and stored as frozen aliquots of 1 ml volume before use. The final concentrations of sodium distearoylphosphatidate and octyl glucoside in the solution were 10 mM and 150 mM, respectively.

The above diagnosis marker (54 µg) in the lyophilized state was added with 10% (v/v) dimethyl sulfoxide (DMSO)/phosphate buffered saline (20 µl, pH 7.4) and dissolved. The above solution of sodium distearoylphosphatidate and octyl glucoside (5 µl) was added to 5 µl of the resulting solution, which was warmed beforehand, and mixed by repeating careful suction and ejection using a micropipette to obtain a composition of the present invention in the form of an aqueous solution. By using an indocyanine green (ICG) solution, ICG-sulfo-OSu solution, and water (control) instead of the solution of the diagnosis marker, aqueous compositions were prepared in the same manner as described above. Fluorescence emission of the above sample solutions (addition groups) and samples that were not added with the solution containing sodium distearoylphosphatidate and octyl glucoside (no addition groups) were observed at 800 nm or more, and increase of fluorescence intensity due to the addition of sodium distearoylphosphatidate was evaluated. In the groups where the solution of sodium distearoylphosphatidate was added to the diagnostic marker, increases of fluorescence were clearly recognized.

BHSM-IR (Olympus Optical) was used as an infrared ray microscope that allows observation in visible light to near infrared light region. The microscope was equipped with an excitation light transmitting filter that transmits lights of 710-790 nm (Asahi Bunko) under the sample, and with an excitation light cutting filter that transmits lights of 810-920 nm (Asahi Bunko) over the sample. KP-MI from Hitachi Electronic Engineering was used as a CCD, and fluorescence caught by the CCD was taken into an image collecting apparatus (EVIP-230, Olympus Optical), and recorded in an image recording apparatus (S321S, Olympus Optical) via an amplification system (Olympus Optical).

5 µl of each of the samples was dropped onto a cotton gauze thread (1 cm) placed on an object glass, and images were observed under irradiation by ordinary light and infrared light using the above optical system. Fluorescence intensity was evaluated using the following criteria: (-) where no fluorescence was observed in the image; (+) where fluorescence was slightly observed; and (++++) where an image was clearly observed, and intermediate levels were evaluated using two criteria of (++) and (+++). The recorded images were converted into final images through an image processing comprising the steps of integration of an original image, integration of a background image, formation of a subtraction image, formation of a filtering image, and contrast enhancement by using the above-mentioned apparatus. The results are shown in Table 1 set out below. In the group where the solution of sodium distearoylphosphatidate was added to the diagnostic marker, increase of fluorescence was clearly recognized. In the table, all of the concentrations indicate those calculated based on ICG concentration. Fig. 1 shows photographs of the cotton gauze threads dropped with the composition of the present invention taken under irradiation by ordinary light and infrared ray. In the figure, (a) represents the result obtained under irradiation of ordinary light, and (b) represents the result obtained under irradiation of infrared ray.

**Table 1**

| Test sample | Concentration in terms of ICG | No addition group | Addition group |
|---|---|---|---|
| ICG | 1 mg/ml | ++ | ++ |
| | 100 µg/ml | - | - |
| | 10 µg/ml | - | - |
| | 1 µg/ml | - | - |
| ICG-sulfo-OSu | 100 µg/ml | ± | ++ |
| | 10 µg/ml | + | + |
| | 1 µg/ml | - | - |
| | 0.1 µg/ml | - | - |
| ICG-sulfo-OSu- | 120 µg/ml | + | +++ |
| labeled antibody | 10 µg/ml | - | ++ |
| | 1 µg/ml | - | + |
| | 0.1 µg/ml | - | ± |
| Water | | - | - |

| | | | |
|---|---|---|---|
| +++: Definitely bright | | | |
| ++: Bright. | | | |
| +: Observable | | | |
| ± Slightly observable | | | |

### Example 2

In the same manner as Example 1, a diagnostic marker comprising mouse anti-mucin antibody (anti-MUC-1: Yamamoto, M., et al., Japanese Journal of Cancer Research, 87(5), pp.488-496, 1996) carrying approximately 16 molecules of ICG-sulfo-OSu per one molecule of the antibody was prepared. The diagnostic marker was stored in frozen state just before use. Absorption spectrum and fluorescence spectrum were measured in the same manner as Example 1, and the maker was found to have the maximum absorption wavelength of 802.8 nm, molar absorption coefficient of 3.90×10⁵ M⁻¹·cm⁻¹ (slit width: 0.2 nm, reference: PBS· containing 37.5 mM of octyl glucoside, measured at ambient temperature); excitation wavelength of 768 nm, and fluorescence wavelength of 820 nm (slit width on the excitation light side: 5.0 nm, slit width on the fluorescence side: 5.0 nm).

A diagnostic marker comprising mouse anti-sulfomucin antibody (91.9H: Irimura, T., et al., Cancer Res., 51, pp.5728-5735, 1991) carrying approximately 16 molecules of ICG-sulfo-OSu per one molecule of the antibody was also prepared. The diagnostic marker was stored in frozen state just before use. Absorption spectrum and fluorescence spectrum were measured in the same manner as Example 1, and the marker was found to have the maximum absorption wavelength of 802.8 nm, molar absorption coefficient of 3.89 × 10⁵ M⁻¹ · cm⁻¹ (slit width: 0.2 nm, reference: PBS· containing 37.5 mM of octyl glucoside, measured at ambient temperature); excitation wavelength of 768 nm, and fluorescence wavelength of 820 nm (slit width on the excitation light side: 5.0 nm, slit width on the fluorescence side: 5.0 nm). Furthermore, by using human anti-CEA antibody, human anti-mucin antibody and human anti-sulfomucin antibody, diagnostic markers comprising each of the antibodies carrying approximately 16 molecules of ICG-sulfo-OSu per one molecule of the antibodies were prepared. These diagnosis markers each gave the same spectrophotometric spectrum data as the diagnostic marker produced using a corresponding mouse antibody.

The above diagnostic marker in the form of lyophilized product obtained from the mouse anti-mucin antibody (100 µg) was added with 4.0 ml of phosphate buffered saline and dissolved. 1.5 ml of the resulting solution was added with 1.5 ml of a solution of the fluorescence intensity enhancing agent of the present invention, and then the mixture was warmed over a water bath at 50°C to prepare a final sample. As a control, a solution was prepared in the same manner by adding 1.5 ml of phosphate buffered saline. The final antibody concentration in the diagnostic marker was 83.3 nM, and the ICG-sulfo-OSu concentration was 1.33 µM.

Fluorescence spectrum of each sample was measured by using a Hitachi spectrophotometer 650-40. The slit width was 5 nm for each of excitation light and fluorescence. Fluorescence intensity of quinine sulfate at 1 ppm was measured as a standard, which was found to be 68.0 (λₑₓ = 255 nm, λₑₘ = 451 nm) before the spectrum measurement, and 69.5 (λₑₓ = 255 nm, λₑₘ = 451 nm) after the spectrum measurement. The results are shown in Table 2.

**Table 2**

| Composition | Fluorescence intensity enhancing agent | Excitation wavelength (nm) | Fluorescence wavelength (nm) | Fluorescence intensity |
|---|---|---|---|---|
| 1^{a} | DSPA + OG | 768 | 825 (807)^{b} | 0.95 (0.19)^{c} |
| 2 | DSPA + OG | 768 | 825 (807) | 1.01 (0.23) |
| 3^{a} | OG | 768 | 820 (807) | 0.92 (0.20) |
| 4 | OG | 768 | 825 (807) | 0.96 (0.25) |
| 5 | DSPC + OG | 768 | 825 (807) | 1.10 (0.26) |
| 6 | DMPA + OG | 768 | 825 (807) | 1.20 (0.29) |
| 7 | STAD + OG | 768 | 825 (807) | 1.17 (0.28) |
| 8 (control) | PBS | 768 | 807 (807) | 0.25 (0.24) |

| | | | | |
|---|---|---|---|---|
| OG : Octyl glucoside (37.5 mM) DSPA: Distearoylphosphatidic acid (2.5 mM) DSPC : Distearoylphosphatidylcholine (2.5 mM) DMPA :Dipalmitoylphosphatidic acid (2.5 mM) STAD : Stearic acid (2.5 mM) PBS : Phosphate buffered saline (control) a : Measured at ambient temperature; as for the other composition, measured immediately after warming at 50°C | | | | |
| b : Parenthesized values indicate the values before the addition of fluorescence intensity enhancing agent. | | | | |
| c : Parenthesized values indicate the values before the addition of fluorescence intensity enhancing agent. | | | | |

Fig. 2 depicts the changes of absorption spectrum and fluorescence spectrum of Composition 6 in the table before and after the addition of dimyristoylphosphatidic acid and octyl glucoside as the fluorescence intensity enhancing agent. In the figure, (c) represents the absorption spectrum before the addition of fluorescence intensity enhancing agent, (c') represents the absorption spectrum after the addition of fluorescence intensity enhancing agent, (d) represents the fluorescence spectrum before the addition of fluorescence intensity enhancing agent, and (d') represents the fluorescence spectrum after the addition of fluorescence intensity enhancing agent. Remarkable increases of intensities of the absorption spectrum and fluorescence spectrum were observed in the composition of the present invention containing the fluorescence intensity enhancing agent. It was recognized that the maximum fluorescence wavelength shifted to the longer wavelength side by about 18 nm in the fluorescence emission spectrum.

### Example 3

To examine the relationship between the fluorescence intensity enhancing effect and concentration of octyl glucoside, a solution was prepared by dissolving 400 µg of the lyophilized ICG-sulfo-OSu labeled anti-mucin antibody, which was prepared in Example 2, in 16.0 ml of phosphate buffered saline, and 1.5 ml aliquots of the resulting solution were added with 1.5 ml of octyl glucoside solutions at various concentrations, and then the mixtures were incubated at 50°C. The final antibody concentration in the diagnostic markers was 83.3 nM, and the ICG-sulfo-OSu concentration was 1.33 µM. Fluorescence spectra of the samples were measured in the same manner as in Example 2. The slit width was 2 nm for each of excitation light and fluorescence. Fluorescence intensity of quinine sulfate at 1 ppm was measured as a standard, which was found to be 100.0 (λₑₓ = 251 nm, λₑₘ = 450 nm) before the spectrum measurement, and 96.0 (λₑₓ = 251 nm, λₑₘ = 450 nm) after the spectrum measurement. The results are shown in Table 3. Sharp increases of fluorescence intensity were observed at concentrations near the critical micelle concentration of octyl glucoside (25 mM), and gradual increases of the fluorescence intensity were observed at higher concentrations. As for the sift of fluorescence wavelength to a longer wavelength region, sharp increases were observed at concentrations near the critical micelle concentration.

**Table 3**

| OG concentration (mM) | Fluorescence intensity | Excitation wavelength (nm) | Fluorescence wavelength (nm) |
|---|---|---|---|
| 0.0 | 0.153 | 768 | 804 |
| 1.0 | 0.150 | 768 | 806 |
| 5.0 | 0.150 | 768 | 804 |
| 10.0 | 0.189 | 768 | 809 |
| 20.0 | 0.436 | 768 | 817 |
| 37.5 | 0.520 | 768 | 817 |
| 50.0 | 0.515 | 768 | 820 |
| 75.0 | 0.525 | 768 | 816 |
| 100 | 0.600 | 768 | 816 |
| 150 | 0.588 | 768 | 821 |
| OG :Octyl glucoside | | | |

### Industrial Applicability

The composition of the present invention is useful for immunohistochemical staining in vivo. For example, by using an infrared ray endoscope or other, the composition is useful for quasi-internal early diagnosis of malignant neoplasia of epithelial tissues such as esophagus cancer, stomach cancer, and large bowel cancer, and identification and diagnosis of lesions during surgical operation. The composition of the present invention is characterized to have excellent fluorescence intensity and does not cause problems of damaging living tissues and DNAs due to irradiation by ultraviolet light, and hence the composition is useful because it enables direct examination or diagnosis reflecting a living state by using an ordinary fluorescence detection apparatus. In particular, because the peak wavelength of fluorescence shifts to the longer wavelength side and absorption intensity at the peak wavelength is markedly increased by using the fluorescence intensity enhancing agent of the present invention. Accordingly, the composition facilitates the observation of fluorescence without interference of excitation light, and remarkably improves allowances of design of the whole measurement system including fluorescence filter.

## Claims

1. A composition for immunohistochemical staining which contains a diagnostic marker comprising an antibody bound with a fluorescent functional group derived from an indocyanine green derivative, and a surfactant consisting of a saccharide derivative selected from the group consisting of octylglucoside, heptylglucoside, octyl thioglucoside, and heptyl thioglucoside.

2. The composition according to claim 1, wherein the composition further comprises a substance selected from the group consisting of a glycerophospholipid or a fatty acid.

3. The composition according to claim 2, wherein the glycerophospholipid is an acylglycerol phosphate.

4. The composition according to claim 3, wherein the acylglycerol phosphate is a 1,2-diacyl-sn-glycerol 3-phosphate containing two C₁₀₋₂₀ fatty acid residues.

5. The composition according to claim 4, wherein the 1,2-diacyl-sn-glycerol 3-phosphate is dimyristoylphosphatidic acid or distearoylphosphatidic acid.

6. The composition according to claim 2, wherein the glycerophospholipid is an acylglycerol phosphocholine.

7. The composition according to claim 6, wherein the acylglycerol phosphocholine is a 1,2-diacyl-sn-glycerol 3-phosphocholine containing two C₁₀₋₂₀ fatty acid residues.

8. The composition according to claim 7, wherein the 1,2-diacyl-sn-glycerol 3-phosphocholine is distearoylphosphatidylcholine.

9. The composition according to claim 1, wherein the surfactant is octyl glucoside.

10. The composition according to any one of claims I to 9, wherein the fluorescent functional group is a functional group derived from indocyanine green-N-hydroxysulfosuccinimide ester.

11. The composition according to any of claims 1 to 10, wherein the antibody is an anti-cancer antigen antibody.

12. A method for immunohistochemically staining a tumor cell by using a composition according to any one of claims 1 to 11.

13. A method for immunohistochemically diagnosing a tumor by using a composition according to any one of claims 1 to 11.

## Patentansprüche

1. Zusammensetzung zum immunhistochemischen Anfärben, die ein diagnostisches Markierungsmittel enthält, umfassend einen Antikörper, der mit einer von einem Derivat des Indocyaningrüns abgeleiteten, fluoreszierenden funktionellen Gruppe verbunden ist, und eine oberflächenaktive Substanz bestehend aus einem Saccharid-Derivat ausgewählt aus der Gruppe bestehend aus Octylglucosid, Heptylglucosid, Octylthioglucosid und Heptylthioglucosid.

2. Zusammensetzung nach Anspruch 1, wobei die Zusammensetzung weiterhin eine Substanz ausgewählt aus der Gruppe bestehend aus einem Glycerophospholipid oder einer Fettsäure umfasst.

3. Zusammensetzung nach Anspruch 2, wobei das Glycerophospholipid ein Acylglycerinphosphat ist.

4. Zusammensetzung nach Anspruch 3, wobei das Acylglycerinphosphat ein 1,2-Diacyl-sn-glycerin-phosphat ist, das zwei C₁₀₋₂₀ Fettsäurereste enthält.

5. Zusammensetzung nach Anspruch 4, wobei das 1,2-Diacyl-sn-glycerin-3-phosphat Dimyristoylphosphatidsäure oder Distearoylphosphatidsäure ist.

6. Zusammensetzung nach Anspruch 2, wobei das Glycerophospholipid ein Acylglycerinphosphocholin ist.

7. Zusammensetzung nach Anspruch 6, wobei das Acylglycerinphosphocholin ein 1,2-Diacyl-sn-glycerin-3-phosphocholin ist, das zwei C₁₀₋₂₀ Fettsäurereste enthält.

8. Zusammensetzung nach Anspruch 7, wobei das 1,2-Diacyl-sn-glycerin-3-phosphocholin Distearoylphosphatidylcholin ist.

9. Zusammensetzung nach Anspruch 1, wobei die oberflächenaktive Substanz Octylglucosid ist.

10. Zusammensetzung nach einem der Ansprüche 1 bis 9, wobei die fluoreszierende funktionelle Gruppe eine von Indocyaningrün-N-hydroxysulfosuccinimidester abgeleitete funktionelle Gruppe ist.

11. Zusammensetzung nach einem der Ansprüche 1 bis 10, wobei der Antikörper ein Anti-Krebs-Antigen-Antikörper ist.

12. Verfahren für das immunhistochemische Anfärben einer Tumorzelle, wobei eine Zusammensetzung nach einem der Ansprüche 1 bis 11 verwendet wird.

13. Verfahren für das immunhistochemische Diagnostizieren eines Tumors, wobei eine Zusammensetzung nach einem der Ansprüche 1 bis 11 verwendet wird.

## Revendications

1. Composition pour la coloration immunohistochimique qui contient un marqueur pour diagnostic comprenant un anticorps lié à un groupe fonctionnel fluorescent dérivant d'un dérivé du vert d'indocyanine, et un tensioactif consistant en un dérivé saccharide sélectionné dans le groupe consistant en un octylglucoside, un heptylglucoside, un octylthioglucoside, et un heptylthioglucoside.

2. Composition selon la revendication 1, dans lequel la composition comprend en outre une substance sélectionnée dans le groupe consistant en un glycérophospholipide et un acide gras.

3. Composition selon la revendication 2, dans lequel le glycérophospholipide est un phosphate d'acylglycérol.

4. Composition selon la revendication 3, dans lequel le phosphate d'acylglycérol est un 1,2-diacyl-sn-glycérol 3-phosphate contenant deux résidus d'acide gras en C₁₀₋₂₀.

5. Composition selon la revendication 4, dans lequel le 1,2-diacyl-sn-glycérol 3-phosphate est l'acide dimirystoylphosphatidique ou l'acide distéaroylphosphatidique.

6. Composition selon la revendication 2, dans laquelle le glycérophospholipide est une acylglycérol phosphocholine.

7. Composition selon la revendication 6, dans laquelle l'acylglycérol phosphocholine est une 1,2-diacyl-sn-glycérol 3-phosphocholine contenant deux résidus d'acide gras en C₁₀₋₂₀.

8. Composition selon la revendication 7, dans laquelle la 1,2-diacyl-sn-glycérol 3-phosphocholine est la distéaroylphosphatidylcholine.

9. Composition selon la revendication 1, dans laquelle le tensioactif est un octylglucoside.

10. Composition selon l'une quelconque des revendications 1 à 9, dans laquelle le groupe fonctionnel fluorescent est un groupe fonctionnel dérivant de l'ester N-hydroxysulfosuccinimide du vert d'indocyanine.

11. Composition selon l'une quelconque des revendications 1 à 10, dans laquelle l'anticorps est un anticorps d'antigène anti-cancer.

12. Procédé de coloration immunohistochimique d'une cellule tumorale par utilisation d'une composition selon l'une quelconque des revendications 1 à 11.

13. Procédé de diagnostic immunohistochimique d'une tumeur par utilisation d'une composition selon l'une quelconque des revendications 1 à 11.
